# FASCICULE DE BREVET EUROPEEN

(11) **EP 1 245 565 B1**
(45) Date de publication et mention de la délivrance du brevet: **24.09.2003**
(21) Numéro de dépôt: 02290668.9
(22) Date de dépôt: 18.03.2002
(51) Int. Cl.: C07D 209/80, A61K 31/40

(54) **Dérivés d'indénoindolones, leur procédé de préparation et les compositions pharmaceutiques qui les contiennent**
Indenoindolon-Derivate, Verfahren zu ihrer Herstellung und die enthaltenden pharmazeutischen Zusammensetzungen
Indenoindolone derivatives, process for their preparation and pharmaceutical compositions containing them

(30) Priorité: 19.03.2001 FR 0103667
(43) Date de publication de la demande: 02.10.2002
(73) Titulaire: Les Laboratoires Servier, 92200 Neuilly-sur-Seine (FR)
(72) Inventeur: Wierzbicki, Michel, 78620 l'Etang La Ville (FR); Boussard, Marie-Francoise, 78124 Mareil sur Mauldre (FR); Rousseau, Anne, 91160 Longjumeau (FR); Boutin, Jean-Albert, 92150 Suresnes (FR); Delagrange, Philippe, 92130 Issy les Moulineaux (FR)

(56) Documents cités:
- DATABASE CHEMABS [en ligne] CHEMICAL ABSTRACTS SERVICE, COLUMBUS, OHIO, US:; CARRUTHERS, WILLIAM ET AL.: "Photolysis of 1-benzoyl- and 1- and 3-o-iodobenzoylindole" XP002177427 & J. CHEM. SOC., PERKIN TRANS. 1, vol. 13, 1974, pages 1523-5,

## Description

La présente invention concerne de nouveaux dérivés d'indénoindolones, leur procédé de préparation, les compositions pharmaceutiques qui les contiennent ainsi que leur utilisation dans le traitement des troubles du système mélatoninergique.

Quelques dérivés d'indénoindolones ont été décrits dans la littérature, notamment dans J. Chem. Soc. Perkin Trans. I 1974, 13, 1523-1525, sans qu'aucune activité pharmacologique n'ait été décrite pour ces composés.

Les composés de la présente invention sont nouveaux et présentent des caractéristiques pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychopharmacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp. 264-272), analgésiques (Pharmacopsychiat., 1987, 20, pp. 222-223), ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986, 24, pp. 359-364), et dans le traitement de l'obésité (International Journal of Eating Disorders, 1996, 20 (4), pp. 443-446).

Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p. 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une forte affinité pour les récepteurs de la mélatonine et une sélectivité importante pour les sites de type MT₃.

Plus particulièrement, la présente invention concerne les composés de formule (I) : 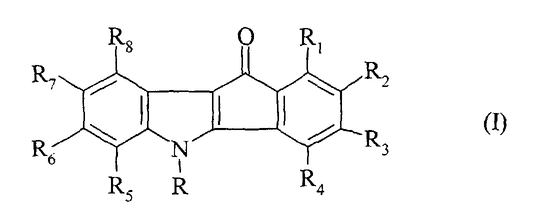 dans laquelle :
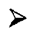 R représente un atome d'hydrogène ou un groupement alkényle (C₁-C₆) linéaire ou ramifié ou alkyle (C₁-C₆) linéaire ou ramifié éventuellement substitué par un groupement carboxy ou par un groupement de formule -NRₐR_{b} dans laquelle Rₐ et R_{b}, identiques ou différents, représentent chacun un atome d'hydrogène ou un groupement alkyle (C₁-C₆) linéaire ou ramifié, ou bien forment ensemble avec l'atome d'azote qui les porte un hétérocycle azoté,
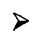 R₁, R₂, R₃, R₄, R₅, R₆, R₇ et R₈, identiques ou différents, représentent chacun un atome d'hydrogène ou un groupement alkyle (C₁-C₆) linéaire ou ramifié, alkényle (C₁-C₆) linéaire ou ramifié, hydroxy, alkoxy (C₁-C₆) linéaire ou ramifié, alkényloxy (C₁-C₆) linéaire ou ramifié, arylalkyle (C₁-C₆) linéaire ou ramifié, arylalkoxy (C₁-C₆) linéaire ou ramifié, carboxy, acyloxy (C₁-C₆) linéaire ou ramifié ou arylcarbonyloxy,
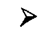 ou bien l'un des groupements R₁ à R₈ forme avec un autre groupement R₁ à R₈ contigu un groupement alkylènedioxy (C₁-C₂),
leurs isomères optiques lorsqu'ils existent, ainsi que leurs sels d'addition à un acide ou à une base pharmaceutiquement acceptable,
à condition que :
* lorsque R ne représente pas un atome d'hydrogène, alors l'un au moins des groupements R₁ à R₈ représente un groupement hydroxy ou acyloxy (C₁-C₆) linéaire ou ramifié,
* et que les composés de formule (I) soient différents de la indéno[1,2-b]indol-10(*5H*)-one.

Par groupement aryle, on entend phényle, biphénylyle, naphtyle, ou tétrahydronaphtyle, chacun de ces groupements étant éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, amino (substitué éventuellement par un ou plusieurs groupements alkyle (C₁-C₆) linéaire ou ramifié), nitro, acyle (C₁-C₆) linéaire ou ramifié et alkylènedioxy (C₁-C₂).

Par hétérocycle azoté, on entend un groupement monocyclique saturé de 5 à 7 chaînons contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre. Les hétérocycles azotés préférés sont les groupements pyrrolidinyle, pipéridyle, morpholinyle ou pipérazinyle.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, méthanesulfonique, camphorique, oxalique, etc.

Parmi les bases pharmaceutiquement acceptables, on peut citer à titre non limitatif l'hydroxyde de sodium, l'hydroxyde de potassium, la triéthylamine, la tertbutylamine, etc.

L'invention s'étend également au procédé de préparation des composés de formule (I) caractérisé en ce que l'on met en réaction un composé de formule (II) : 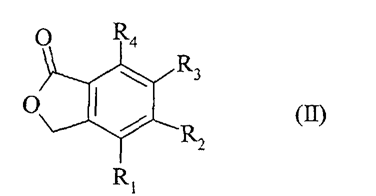 dans laquelle R₁, R₂, R₃ et R₄ ont la même signification que dans la formule (I),
avec le N-bromosuccinimide pour conduire au composé de formule (III) : 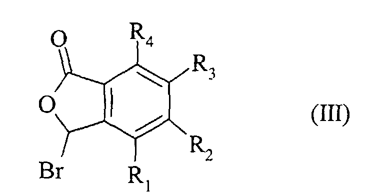 dans laquelle R₁, R₂, R₃ et R₄ ont la même signification que précédemment,
que l'on met en réaction avec la triphénylphosphine pour conduire au composé de formule (IV) : 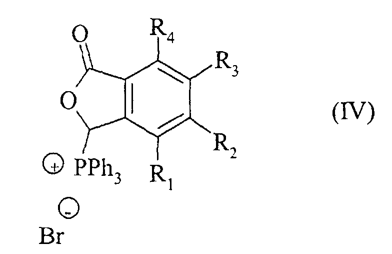 dans laquelle R₁, R₂, R₃ et R₄ ont la même signification que précédemment,
que l'on met en réaction avec un composé de formule (V) : 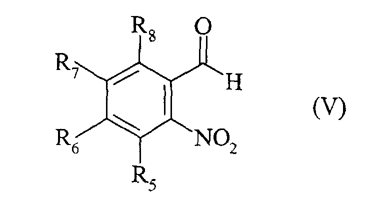 dans laquelle R₅, R₆, R₇ et R₈ ont la même signification que dans la formule (I),
pour conduire au composé de formule (VI) : 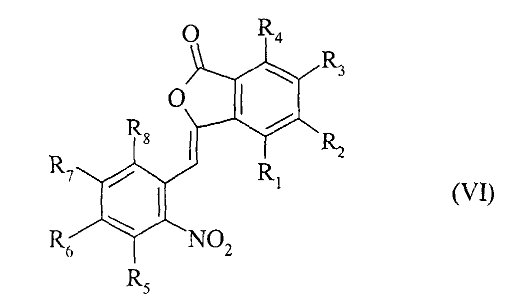 dans laquelle R₁, R₂, R₃, R₄, R₅, R₆, R₇ et R₈ ont la même signification que précédemment,
que l'on met en présence de base pour conduire au composé de formule (VII) : 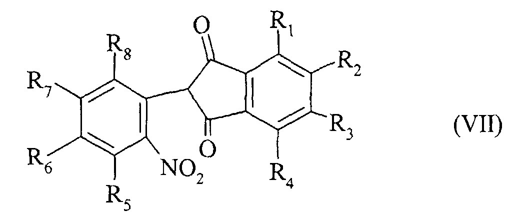 dans laquelle R₁, R₂, R₃, R₄, R₅, R₆, R₇ et R₈ ont la même signification que précédemment,
que l'on soumet à un agent de réduction pour conduire, après séparation des isomères le cas échéant, au composé de formule (la), cas particulier des composés de formule (I) : 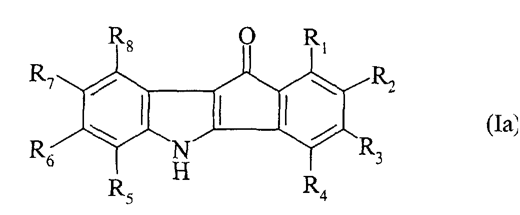 dans laquelle R₁, R₂, R₃, R₄, R₅, R₆, R₇ et R₈ ont la même signification que précédemment,
que l'on met en réaction, lorsqu'on le souhaite, avec un composé de formule (VIII) :

R'-Z (VIII)

dans laquelle R' représente un groupement alkényle (C₁-C₆) linéaire ou ramifié ou alkyle (C₁-C₆) linéaire ou ramifié éventuellement substitué par un groupement carboxy ou par un groupement de formule -NRₐR_{b} dans laquelle Rₐ et R_{b}, identiques ou différents, représentent chacun un atome d'hydrogène ou un groupement alkyle (C₁-C₆) linéaire ou ramifié, ou bien forment ensemble avec l'atome d'azote qui les porte un hétérocycle azoté, et Z représente un groupement partant tel que, par exemple, un atome d'halogène ou un groupement mésylate, tosylate ou trifluorométhanesulfonate,
pour conduire au composé de formule (Ib), cas particulier des composés de formule (I) : 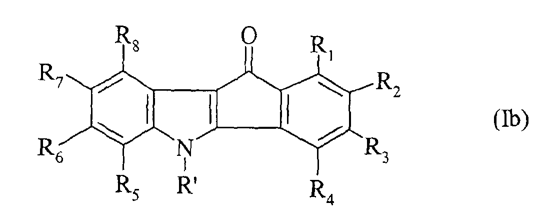 dans laquelle R₁, R₂, R₃, R₄, R₅, R₆, R₇, R₈ et R' ont la même signification que précédemment,
composés de formule (Ia) ou (Ib) qui constituent l'ensemble des composés de formule (I), que l'on purifie, le cas échéant, selon une technique classique de purification, dont on sépare, le cas échéant, les isomères selon une technique classique de séparation, et que l'on transforme, si on le souhaite, en leurs sels d'addition à un acide ou à une base pharmaceutiquement acceptable.

Les composés de formule (I) pour lesquels l'un des groupements R₁ à R₈ représente un groupement hydroxy peuvent également être obtenus par hydrogénation catalytique des éthers de benzyle correspondants ou par déméthylation des éthers méthyliques correspondants.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une haute affinité sélective pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation, qui permettent d'établir que les produits de l'invention sont utiles dans le traitement du stress, des troubles du sommeil, de l'anxiété, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immmunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité. Par exemple, les composés seront utilisés dans le traitement des dépressions saisonnières et des troubles du sommeil.

La présente invention a également pour objet les compositions pharmaceutiques contenant un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon.

Les produits de départ utilisés sont des produits connus ou préparés selon des modes préparatoires connus.

Les structures des composés décrits dans les exemples ont été déterminées selon les techniques spectrométriques usuelles (infrarouge, RMN, spectrométrie de masse).

### EXEMPLE 1 : 8-Benzyloxy-2,3-diméthoxyindéno[1,2-b]indol-10(5H)-one

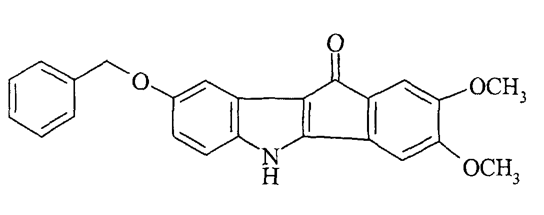

### Stade A : 3 -Bromo-5,6-diméthoxy -phtalide

A 10 mmoles de 5,6-diméthoxy-phtalide en solution dans le dichlorométhane sont ajoutées 12 mmoles de N-bromo-succinimide, puis le mélange réactionnel, éclairé par une lampe halogène, est porté au reflux pendant 5 heures. Le mélange est ensuite ramené à température ambiante et filtré, puis le filtrat est évaporé, du toluène est ajouté, la suspension obtenue est filtrée et le filtrat est évaporé. Le résidu obtenu est filtré sur silice pour conduire au produit attendu.

### Stade B : Bromure de (5,6-diméthoxy-phtalidyl)-triphénylphosphonium

A 10 mmoles du composé obtenu dans le stade précédent en solution dans le toluène sont ajoutées 10 mmoles de triphénylphosphine, puis le mélange réactionnel est porté au reflux pendant 3 heures. Après retour à température ambiante, le mélange est filtré puis le gâteau obtenu est lavé et séché pour conduire au produit attendu.
*Point de fusion : > 260°C*

### Stade C : 3-(5-Benzyloxy-2-nitro-benzylidène)-5,6-diméthoxy-phtalide

A 10 mmoles de 5-benzyloxy-2-nitro-benzaldéhyde en solution dans le diméthylformamide sont ajoutées 10 mmoles de triéthylamine, puis, par portions, 10 mmoles du composé obtenu dans le stade précédent. Le mélange réactionnel est ensuite chauffé à 50°C pendant 1 heure 30, puis ramené à température ambiante et évaporé. De l'éther est ensuite ajouté, le mélange est agité pendant une nuit, puis filtré. Le gâteau obtenu est ensuite lavé pour conduire au produit attendu.
*Point de fusion : 170°C*

### Stade D : 2 - (5-Benzyloxy-2-nitrophényl) -3-hydroxy-5,6-diméthoxy-1H-inden-1-one

A 10 mmoles du composé obtenu dans le stade précédent en solution dans le méthanol sont ajoutés 10 ml d'une solution 4N d'hydroxyde de sodium. Le mélange est ensuite porté à 40°C pendant 1 heure, puis refroidi à 0°C et amené à pH = 1 avec une solution d'acide chlorhydrique 4N (12 ml). Après une nuit d'agitation à température ambiante, le précipité blanc formé est filtré, lavé puis séché et purifié par chromatographie sur colonne de silice (éluant : dichlorométhane/méthanol 99/1) pour conduire au produit attendu.
*Point de fusion : 120°C*

### Stade E : 8-Benzyloxy-2,3-diméthoxyindéno[1,2-b]indol-10(5H)-one

Une solution du composé obtenu dans le stade précédent (10 mmoles) dans le diméthylformamide est placée sous hydrogène en présence de nickel de Raney. Après filtration du catalyseur, le solvant est évaporé et le résidu est séché pour conduire au produit attendu.
*Point de fusion : 235°C*

### EXEMPLE 2 : 2,3-Diméthoxy-8-hydroxy-indéno[1,2-b]indol-10(5H)-one

A une solution du composé décrit dans l'exemple 1 (10 mmoles) dans un mélange méthanol/diméthylformamide 70/30 sont ajoutées 20 mmoles d'une solution 1N d'hydroxyde de sodium, puis 1 g de charbon palladié à 10 %. Le mélange est ensuite placé sous une pression d'hydrogène de 80 mbars pendant 2 heures à température ambiante, puis le catalyseur est filtré, une solution d'acide chlorhydrique 1N (20 ml) est ajoutée au filtrat, et les solvants sont évaporés. Le résidu obtenu est ensuite repris par de l'isopropanol, puis la suspension obtenue est filtrée, le filtrat est évaporé et le résidu obtenu est lavé pour conduire au produit attendu.
*Point de fusion : >260°C*

### EXEMPLE 3 : 2,3-Diméthoxy-8-hydroxy-5-méthyl-indéno[1,2-b]indol-10(5H)-one

A une solution du composé décrit dans l'exemple 2 (10 mmoles) dans le diméthylformamide sont ajoutées 11 mmoles de carbonate de potassium, puis le mélange réactionnel est amené à 90°C et 11 mmoles d'iodométhane sont ajoutées. Le milieu réactionnel est ensuite maintenu à 60°C pendant 1 nuit, puis le solvant est évaporé, de l'eau est ajoutée et la suspension obtenue est filtrée. Le gâteau est ensuite recristallisé dans l'éthanol pour conduire au produit attendu.
*Point de fusion : 241°C*

| *Microanalyse élémentaire* : | | | |
|---|---|---|---|
| | *C%* | *H%* | *N%* |
| *calculé* | *68,89* | *4,89* | *4,53* |
| *trouvé* | *69,04* | *4,81* | *4,61* |

### EXEMPLE 4 : 7-Benzyloxy-8-méthoxy-indéno[1,2-b]indol-10(5H)-one

Le produit attendu est obtenu selon le procédé décrit dans l'exemple 1 à partir de phtalide et de 4-benzyloxy-5-méthoxy-2-nitro-benzaldéhyde.

### EXEMPLE 5 : 7-Hydroxy-8-méthoxy-indéno[1,2-b]indol-10(5H)-one

Le produit attendu est obtenu selon le procédé décrit dans l'exemple 2 à partir du composé décrit dans l'exemple 4.
*Point de fusion : >260°C*

| *Microanalyse élémentaire :* | | | |
|---|---|---|---|
| | *C%* | *H%* | *N%* |
| *calculé* | *72,45* | *4,18* | *5,28* |
| *trouvé* | *72,20* | *4,06* | *5,31* |

### EXEMPLE 6 : 8-Benzyloxy-1,4-diméthoxyindéno[1,2-b]indol-10(5H)-one

Le produit attendu est obtenu selon le procédé décrit dans l'exemple 1 à partir de 4,7-diméthoxy-phtalide et de 5-benzyloxy-2-nitro-benzaldéhyde.

### EXEMPLE 7 : 1,4-Diméthoxy-8-hydroxy-indéno[1,2-b]indol-10(5H)-one

Le produit attendu est obtenu selon le procédé décrit dans l'exemple 2 à partir du composé décrit dans l'exemple 6.
*Point de fusion : >260°C*

### EXEMPLE 8 : 1,4-Diméthoxy-8-hydroxy-5-méthyl-indéno[1,2-b]indol-10(5H)-one

Le produit attendu est obtenu selon le procédé décrit dans l'exemple 3 à partir du composé décrit dans l'exemple 7.

### EXEMPLE 9 : 8-Benzyloxy-1-hydroxy-4-méthoxy-indéno[1,2-b]indol-10(5H)-one

Le produit attendu est obtenu par déméthylation sélective du composé décrit dans l'exemple 6, selon le procédé décrit dans Tet. 1996, 52 (43), 13623-640.

### EXEMPLE 10 : 1,8-Dihydroxy-4-méthoxy-indéno[1,2-b]indol-10(5H)-one

Le produit attendu est obtenu selon le procédé décrit dans l'exemple 2 à partir du composé décrit dans l'exemple 9.

### EXEMPLE 11 : 2,3-Diméthoxy-indéno[1,2-b]indol-10(5H)-one

Le produit attendu est obtenu selon le procédé décrit dans l'exemple 1 à partir de 5,6-diméthoxy-phtalide et de 2-nitro-benzaldéhyde.
*Point de fusion : 260°C*

### EXEMPLE 12 : 2,3-Diméthoxy-7,8-méthylènedioxy-indéno[1,2-b]indol-10(5H)-one

Le produit attendu est obtenu selon le procédé décrit dans l'exemple 1 à partir de 5,6-diméthoxy-phtalidc et de 4,5-méthylènedioxy-2-nitro-benzaldéhyde.

### EXEMPLE 13 : 7,8-Méthylènedioxy-indéno[1,2-b]indol-10(5H)-one

Le produit attendu est obtenu selon le procédé décrit dans l'exemple 1 à partir de phtalide et de 4,5-méthylènedioxy-2-nitro-benzaldéhyde.

### EXEMPLE 14 : 8-Benzyloxy-1,3-diméthoxy-indéno[1,2-b]indol-10(5H)-one

En opérant selon le procédé décrit dans l'exemple 1 à partir de 4,6-diméthoxy-phtalide et de 5-benzyloxy-2-nitro-benzaldéhyde, on obtient le produit attendu en mélange avec la 8-benzyloxy-2,4-diméthoxy-indéno[ 1,2-b]indol-10(5H)-one.

Le produit attendu est obtenu par séparation de ce mélange par chromatographie sur colonne de silice (éluant dichlorométhane/méthanol 98/2), en évaporant la deuxième fraction par ordre d'élution.

### EXEMPLE 15 : 1,3-Diméthoxy-8-hydroxy-indéno[1,2-b]indol-10(5H)-one

Le produit attendu est obtenu selon le procédé décrit dans l'exemple 2 à partir du composé décrit dans l'exemple 14.

### EXEMPLE 16 : 8-Benzyloxy-2,4-diméthoxy-indéno[1,2-b]indol-10(5H)-one

En opérant selon le procédé décrit dans l'exemple 1 à partir de 4,6-diméthoxy-phtalide et de 5-benzyloxy-2-nitro-benzaldéhyde, on obtient le produit attendu en mélange avec la 8-benzyloxy-1,3-diméthoxy-indéno[1,2-b]indol-10(5H)-one.

Le produit attendu est obtenu par séparation de ce mélange par chromatographie sur colonne de silice (éluant dichlorométhane/méthanol 98/2), en évaporant la première fraction par ordre d'élution.

### EXEMPLE 17 : 2,4-Diméthoxy-8-hydroxy-indéno[1,2-b]indol-10(5H)-one

Le produit attendu est obtenu selon le procédé décrit dans l'exemple 2 à partir du composé décrit dans l'exemple 16.

### EXEMPLE 18 : 5-Allyle-2,3-diméthoxy-8-hydroxy-indéno[1,2-b]indol-10(5H)-one

Le produit attendu est obtenu selon le procédé décrit dans l'exemple 3 à partir du composé décrit dans l'exemple 2, en remplaçant l'iodométhane par le bromure d'allyle.

### EXEMPLE 19 : 1,3-Diméthoxy -5 - (2 -diméthylaminoéthyl ) -8 -hydroxy-indéno[1,2-b] indol-10(5H)-one

Le produit attendu est obtenu selon le procédé décrit dans l'exemple 3 à partir du composé décrit dans l'exemple 15, en remplaçant l'iodométhane par la 2-chloro-N,N-diméthyléthanamine.

### EXEMPLE 20 : 1,4-Diméthoxy-5-(2-diméthylaminoéthyl)-8-hydroxy-indéno[1,2-b] indol-10(5H)-one

Le produit attendu est obtenu selon le procédé décrit dans l'exemple 3 à partir du composé décrit dans l'exemple 7, en remplaçant l'iodométhane par la 2-chloro-N,N-diméthyléthanamine.

### EXEMPLE 21 : 2,3-Diméthoxy-5-(2-diméthylaminoéthyl)-8-hydroxy-indéno[1,2-b] indol-10(5H)-one

Le produit attendu est obtenu selon le procédé décrit dans l'exemple 3 à partir du composé décrit dans l'exemple 2, en remplaçant l'iodométhane par la 2-chloro-N,N-diméthyléthanamine.
*Point de fusion : 216°C*

| *Microanalyse élémentaire :* | | | |
|---|---|---|---|
| | *C%* | *H%* | *N%* |
| *calculé* | *68,84* | *6,05* | *7,65* |
| *trouvé* | *68,64* | *5,98* | *7,58* |

### EXEMPLE 22 : 2,3-Diméthoxy-8-hydroxy-5-[2-(4-morpholinyl)-éthyl]-indéno[1,2-b] indol-10(5H)-one

Le produit attendu est obtenu selon le procédé décrit dans l'exemple 3 à partir du composé décrit dans l'exemple 2, en remplaçant l'iodométhane par la 4-(2-chloroéthyl)-morpholine.
*Point de fusion : 228-232°C*

| *Microanalyse élémentaire :* | | | |
|---|---|---|---|
| | *C%* | *H%* | *N%* |
| *calculé* | *67,63* | *5,92* | *6,86* |
| *trouvé* | *67,19* | *5,93* | *6,80* |

### EXEMPLE 23 : 2,3,8-Triméthoxy-indéno[1,2-b]indol-10(5H)-one

Le produit attendu est obtenu selon le procédé décrit dans l'exemple 1 en remplaçant le 5-benzyloxy-2-nitro-benzaldéhyde par le 5-méthoxy-2-nitrobenzaldéhyde.

### EXEMPLE 24: Sel de sodium de l'acide (8-hydroxy-2,3-diméthoxy-10-oxoindéno[1,2-b] indol-5 (10H)-yl) acétique :

### Stade A : (8-Hydroxy-2,3-diméthoxy-10-oxoindéno[1,2-b] indol-5 (10H)-yl) acétate de méthyle

Le produit attendu est obtenu selon le procédé décrit dans l'exemple 3 à partir du composé décrit dans l'exemple 2, en remplaçant l'iodométhane par le chloroacétate de méthyle.

### Stade B : Sel de sodium de l'acide (8 -hydroxy -2,3 -diméthoxy -10 -oxoindéno[1,2-b] indol-5 (10H)-yl) acétique :

Le produit attendu est obtenu par saponification de l'ester obtenu au stade A précédent par la soude.
*Point de fusion : > 260°C*

| *Microanalyse élémentaire :* | | | |
|---|---|---|---|
| | *C%* | *H%* | *N%* |
| *calculé* | *60,80* | *3,76* | *3,73* |
| *trouvé* | *60,38* | *3,77* | *3,74* |

### Etude pharmacologique des composés de l'invention

### EXEMPLE 25 : Etude de liaison aux sites de liaisons MT₃ de la mélatonine

La liaison sur les sites MT₃ est caractérisée par une cinétique d'association et de dissociation remarquablement rapide et par sa localisation tissulaire (cerveaux).
Les expériences de liaison sur les sites MT₃ sont réalisées sur membranes de cerveau de hamster en utilisant la 2-[¹²⁵I] iodomélatonine comme radioligand suivant le protocole décrit par P. Paul et coll. (J. Pharmacol. Exp. Ther. 1999, 290, 334). Les membranes sont incubées pendant 30 minutes avec la 2-[¹²⁵I] iodomélatonine à la température de 4°C et différentes concentrations des composés à tester. Après l'incubation, les membranes sont rapidement filtrées puis lavées par du tampon froid à l'aide d'un système de filtration. La radioactivité fixée est mesurée par un compteur à scintillation.

Les valeurs d'IC₅₀ trouvées pour les composés de l'invention attestent d'une forte affinité pour les sites de type MT₃, ces valeurs étant comprises entre 0,2 et 100 nM. A titre de comparaison, la mélatonine présente une IC₅₀ de 45 nM dans ce test.

### EXEMPLE 26 : Composition pharmaceutique

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 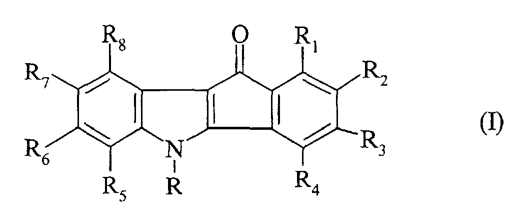 dans laquelle :
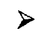 R représente un atome d'hydrogène ou un groupement alkényle (C₁-C₆) linéaire ou ramifié ou alkyle (C₁-C₆) linéaire ou ramifié éventuellement substitué par un groupement carboxy ou par un groupement de formule -NRₐR_{b} dans laquelle Rₐ et R_{b}, identiques ou différents, représentent chacun un atome d'hydrogène ou un groupement alkyle (C₁-C₆) linéaire ou ramifié, ou bien forment ensemble avec l'atome d'azote qui les porte un hétérocycle azoté,
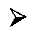 R₁, R₂, R₃, R₄, R₅, R₆, R₇ et R₈, identiques ou différents, représentent chacun un atome d'hydrogène ou un groupement alkyle (C₁-C₆) linéaire ou ramifié, alkényle (C₁-C₆) linéaire ou ramifié, hydroxy, alkoxy (C₁-C₆) linéaire ou ramifié, alkényloxy (C₁-C₆) linéaire ou ramifié, arylalkyle (C₁-C₆) linéaire ou ramifié, arylalkoxy (C₁-C₆) linéaire ou ramifié, carboxy, acyloxy (C₁-C₆) linéaire ou ramifié ou arylcarbonyloxy,
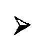 ou bien l'un des groupements R₁ à R₈ forme avec un autre groupement R₁ à R₈ contigu un groupement alkylènedioxy (C₁-C₂),
leurs isomères optiques lorsqu'ils existent, ainsi que ses sels d'addition à un acide ou à une base pharmaceutiquement acceptable, à condition que :
* lorsque R ne représente pas un atome d'hydrogène, alors l'un au moins des groupements R₁ à R₈ représente un groupement hydroxy ou acyloxy (C₁-C₆) linéaire ou ramifié,
* et que les composés de formule (I) soient différents de la indéno[1,2-b]indol-10(5*H*)-one,
étant entendu que par groupement aryle, on entend phényle, biphénylyle, naphtyle, ou tétrahydronaphtyle, chacun de ces groupements étant éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, amino (substitué éventuellement par un ou plusieurs groupements alkyle (C₁-C₆) linéaire ou ramifié), nitro, acyle (C₁-C₆) linéaire ou ramifié et alkylènedioxy (C₁-C₂).

2. Composé de formule (I) selon la revendication 1 qui est la 2,3-diméthoxy-8-hydroxy-5-méthyl-indéno[1,2-b]indol-10(5*H*)-one.

3. Composé de formule (I) selon la revendication 1 qui est la 2,4-diméthoxy-8-hydroxyindéno[1,2-b]indol-10(5*H*)-one.

4. Composé de formule (I) selon la revendication 1 qui est la 2,3-diméthcxy-5-(2-diméthylaminoéthyl)-8-hydroxy-indéno[1,2-b]indol-10(5*H*)-one.

5. Composé de formule (I) selon la revendication 1 qui est la 2,3-Diméthoxy-8-hydroxy-5-[2-(4-morpholinyl)-éthyl]-indéno[1,2-b]indol-10(5*H*)-one.

6. Procédé de préparation des composés de formule (I) selon la revendication 1 **caractérisé en ce que** l'on met en réaction un composé de formule (II) : 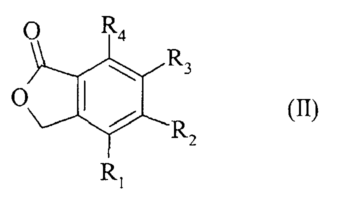 dans laquelle R₁, R₂, R₃ et R₄ ont la même signification que dans la formule (I),
avec le N-bromosuccinimide pour conduire au composé de formule (III) : 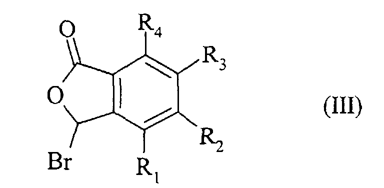 dans laquelle R₁, R₂, R₃ et R₄ ont la même signification que précédemment,
que l'on met en réaction avec la triphénylphosphine pour conduire au composé de formule (IV) : 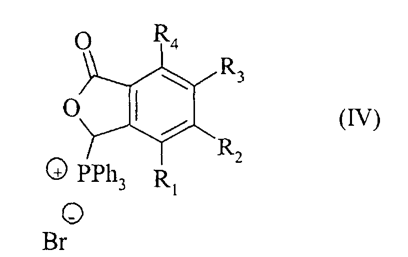 dans laquelle R₁, R₂, R₃ et R₄ ont la même signification que précédemment,
que l'on met en réaction avec un composé de formule (V) : 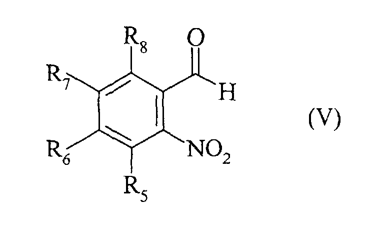 dans laquelle R₅, R₆, R₇ et R₈ ont la même signification que dans la formule (I),
pour conduire au composé de formule (VI) : 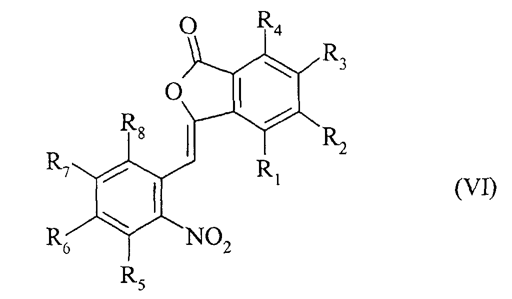 dans laquelle R₁, R₂, R₃, R₄, R₅, R₆, R₇ et R₈ ont la même signification que précédemment,
que l'on met en présence de base pour conduire au composé de formule (VII) : 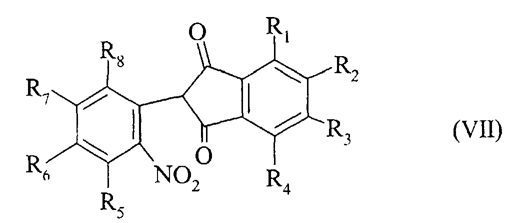 dans laquelle R₁, R₂, R₃, R₄, R₅, R₆, R₇ et R₈ ont la même signification que précédemment,
que l'on soumet à un agent de réduction pour conduire, après séparation des isomères le cas échéant, au composé de formule (Ia), cas particulier des composés de formule (I) : 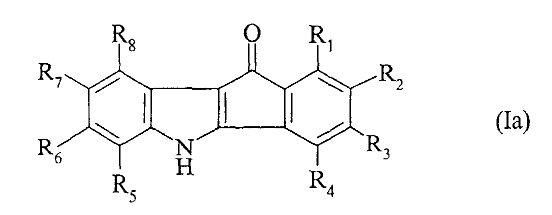 dans laquelle R₁, R₂, R₃, R₄, R₅, R₆, R₇ et R₈ ont la même signification que précédemment,
que l'on met en réaction, lorsqu'on le souhaite, avec un composé de formule (VIII) :
R'-Z (VIII)
dans laquelle R' représente un groupement alkényle (C₁-C₆) linéaire ou ramifié ou alkyle (C₁-C₆) linéaire ou ramifié éventuellement substitué par un groupement carboxy ou par un groupement de formule -NRₐR_{b} dans laquelle Rₐ et R_{b}, identiques ou différents, représentent chacun un atome d'hydrogène ou un groupement alkyle (C₁-C₆) linéaire ou ramifié, ou bien forment ensemble avec l'atome d'azote qui les porte un hétérocycle azoté, et Z représente un groupement partant tel que, par exemple, un atome d'halogène ou un groupement mésylate, tosylate ou trifluorométhanesulfonate,
pour conduire au composé de formule (Ib), cas particulier des composés de formule (I) : 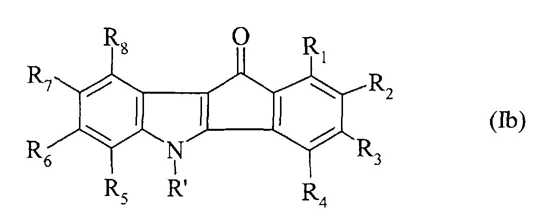 dans laquelle R₁, R₂, R₃, R₄, R₅, R₆, R₇, R₈ et R' ont la même signification que précédemment,
composés de formule (Ia) ou (Ib) qui constituent l'ensemble des composés de formule (I), que l'on purifie, le cas échéant, selon une technique classique de purification, dont on sépare, le cas échéant, les isomères selon une technique classique de séparation, et que l'on transforme, si on le souhaite, en leurs sels d'addition à un acide ou à une base pharmaceutiquement acceptable.

7. Composition pharmaceutique contenant comme principe actif un composé de formule (I) selon l'une quelconque des revendications 1 à 5, en combinaison avec un ou plusieurs véhicules inertes, non toxiques et pharmaceutiquement acceptables.

8. Composition pharmaceutique selon la revendication 7 utile pour la fabrication de médicaments pour traiter les troubles du système mélatoninergique.

## Patentansprüche

1. Verbindung der Formel (I): 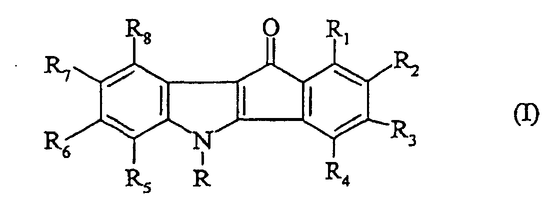 in der:
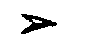 R ein Wasserstoffatom oder eine geradkettige oder verzweigte (C₁-C₆)-Alkenylgruppe oder geradkettige oder verzweigte (C₁-C₆)-Alkylgruppe, die gegebenenfalls durch eine Carboxygruppe oder eine Gruppe der Formel -NRₐR_{b}, in der Rₐ und R_{b}, die gleichartig oder verschieden sind, jeweils ein Wasserstoffatom oder eine geradkettige oder verzweigte (C₁-C₆)-Alkylgruppe bedeuten oder gemeinsam mit dem sie tragenden Stickstoffatom einen Stickstoff-haltigen Heterocyclus bilden, substituiert ist, bedeutet,
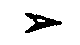 R₁, R₂, R₃, R₄, R₅, R₆, R₇ und R₈, die gleichartig oder verschieden sind, jeweils ein Wasserstoffatom oder eine geradkettige oder verzweigte (C₁-C₆)-Alkylgruppe, geradkettige oder verzweigte (C₁-C₆)-Alkenylgruppe, Hydroxygruppe, geradkettige oder verzweigte (C₁-C₆)-Alkoxygruppe, geradkettige oder verzweigte (C1-C₆)-Alkenyloxygruppe, geradkettige oder verzweigte Aryl-(C₁-C₆)-alkylgruppe, geradkettige oder verzweigte Aryl-(C₁-C₆)-alkoxygruppe, Carboxygruppe, geradkettige oder verzweigte (C₁-C₆)-Acyloxygruppe oder Arylcarbonyloxygruppe bedeuten,
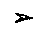 oder eine der Gruppen R₁ bis R₈ mit einer anderen angrenzenden Gruppe R₁ bis R₈ eine (C₁-C₆)-Alkylendioxygruppe bildet,
deren optische Isomeren, falls diese existieren, sowie seine Additionssalze mit einer pharmazeutisch annehmbaren Säure oder Base,
mit der Maßgabe, daß:
* wenn R kein Wasserstoffatom darstellt, dann mindestens eine der Gruppen R₁ bis R₈ eine Hydroxygruppe oder geradkettige oder verzweigte (C₁-C₆)-Acyloxygruppe bedeutet,
* und die Verbindungen der Formel (I) von Indeno[1,2-b]indol-10(5*H*)-on verschieden sind,
mit der Maßgabe, daß man unter einer Arylgruppe Phenyl, Biphenyl, Naphthyl oder Tetrahydronaphthyl versteht, wobei jede dieser Gruppen gegebenenfalls durch ein oder mehrere gleichartige oder verschiedenartige Atome oder Gruppen ausgewählt aus Halogenatomen und geradkettigen oder verzweigten (C₁-C₆)-Alkylgruppen, Hydroxygruppen, geradkettigen oder verzweigten (C₁-C₆)-Alkoxygruppen, geradkettigen oder verzweigten (C₁-C₆)-Polyhalogenalkylgruppen, Aminogruppen (die gegebenenfalls durch eine oder mehrere geradkettige oder verzweigte (C₁-C₆)-Alkylgruppen substituiert sind), Nitrogruppen, geradkettigen oder verzweigten (C₁-C₆)-Acylgruppen und (C₁-C₆)-Alkylendioxygruppen substituiert ist.

2. Verbindung der Formel (I) nach Anspruch 1, nämlich 2,3-Dimethoxy-8-hydroxy-5-methyl-indeno[1,2-b]indol-10(5*H*)-on.

3. Verbindung der Formel (I) nach Anspruch 1, nämlich 2,4-Dimethoxy-8-hydroxy-indeno[1,2-b]indol-10(5*H*)-on.

4. Verbindung der Formel (I) nach Anspruch 1, nämlich 2,3-Dimethoxy-5-(2-dimethylaminoethyl)-8-hydroxy-indeno[1,2-b]indol-10(5*H*)-on.

5. Verbindung der Formel (I) nach Anspruch 1, nämlich 2,3-Dimethoxy-8-hydroxy-5-[2-(4-morpholinyl)-ethyl]-indeno[1,2-b]indol-10(5*H*)-on.

6. Verfahren zur Herstellung der Verbindungen der Formel (I) nach Anspruch 1, **dadurch gekennzeichnet, daß** man eine Verbindung der Formel (II): 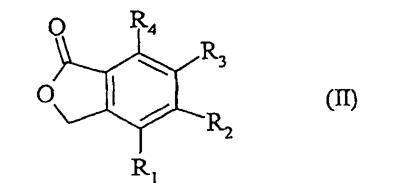 in der R₁, R₂, R₃ und R₄ die bezüglich der Formel (I) angegebenen Bedeutungen besitzen,
mit N-Bromsuccinimid umsetzt zur Bildung der Verbindung der Formel (III): 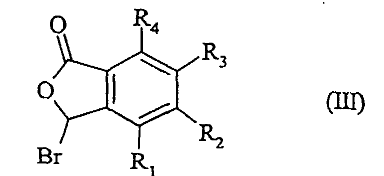 in der R₁, R₂, R₃ und R₄ die oben angegebenen Bedeutungen besitzen,
welche man mit Triphenylphosphin umsetzt zur Bildung der Verbindung der Formel (IV): 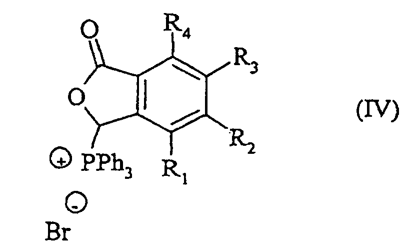 in der R₁, R₂, R₃ und R₄ die oben angegebenen Bedeutungen besitzen,
welche man mit einer Verbindung der Formel (V) umsetzt: 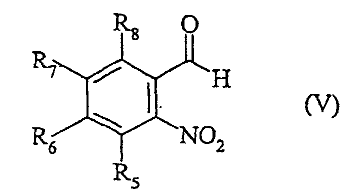 in der R₅, R₆, R₇ und R₈ die bezüglich der Formel (I) angegebenen Bedeutungen besitzen,
zur Bildung der Verbindung der Formel (VI): 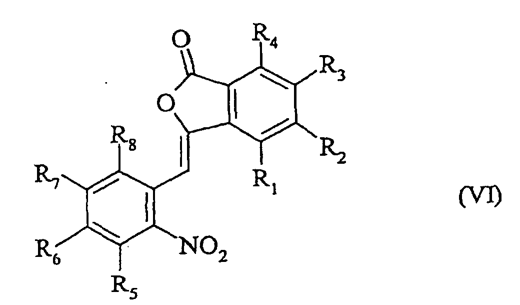 in der R₁, R₂, R₃, R₄, R₅, R₆, R₇ und R₈ die oben angegebenen Bedeutungen besitzen,
welche man mit einer Base umsetzt zur Bildung der Verbindung der Formel (VII): 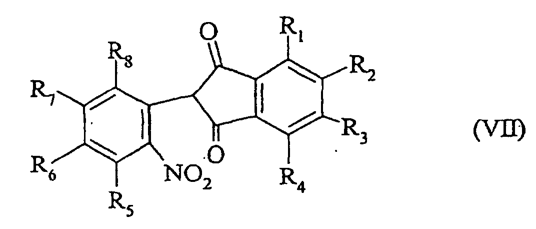 in der R₁, R₂, R₃, R₄, R₅, R₆, R₇ und R₈ die oben angegebenen Bedeutungen besitzen,
welche man der Einwirkung einer Reduktionsmittels unterwirft, so daß man nach der Trennung der gegebenenfalls vorliegenden Isomeren die Verbindung der Formel (Ia) erhält, einem Sonderfall der Verbindungen der Formel (I): 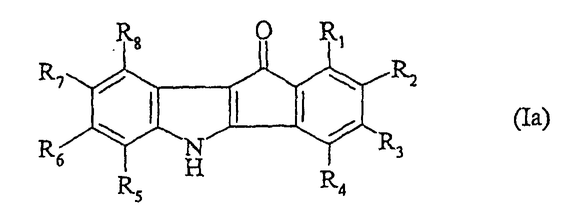 in der R₁, R₂, R₃, R₄, R₅, R₆, R₇ und R₈ die oben angegebenen Bedeutungen besitzen,
welche man gewünschtenfalls mit einer Verbindung der Formel (VIII) umsetzt:
R' - Z (VIII)
in der R' eine geradkettige oder verzweigte (C₁-C₆)-Alkenylgruppe oder geradkettige oder verzweigte (C₁-C₆)-Alkylgruppe, die gegebenenfalls durch eine Carboxygruppe oder eine Gruppe der Formel -NRₐR_{b} substituiert ist, in der Rₐ und R_{b}, die gleichartig oder verschieden sind, jeweils ein Wasserstoffatom oder eine geradkettige oder verzweigte (C₁-C₆)-Alkylgruppe bedeuten oder gemeinsam mit dem sie tragenden Stickstoffatom einen Stickstoff-haltigen Heterocyclus bilden, und Z eine austretende Gruppe, wie beispielsweise ein Halogenatom oder eine Mesylat-, Tosylat- oder Trifluormethansulfonat-gruppe bedeuten,
zur Bildung der Verbindung der Formel (Ib), einem Sonderfall der Verbindungen der Formel (I): 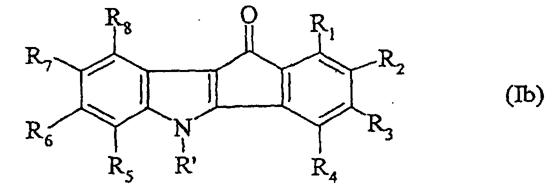 in der R₁, R₂, R₃, R₄, R₅, R₆, R₇, R₈ und R' die oben angegebenen Bedeutungen besitzen,
welche Verbindungen der Formel (Ia) oder (Ib), welche die Gesamtheit der Verbindungen der Formel (I) bilden, man gegebenenfalls mit Hilfe einer klassischen Reinigungsmethode reinigt, gegebenenfalls mit Hilfe einer klassischen Trennungsmethode In ihre Isomeren trennt und gewünschtenfalls in ihre Additionssalze mit einer pharmazeutisch annehmbaren Säure oder Base umwandelt.

7. Pharmazeutische Zubereitung enthaltend als Wirkstoff eine Verbindung der Formel (I) nach einem der Ansprüche 1 bis 5 in Kombination mit einem oder mehreren inerten, nichttoxischen und pharmazeutisch annehmbaren Trägermaterialien.

8. Pharmazeutische Zubereitung nach Anspruch 7 für die Herstellung von Arzneimitteln zur Behandlung von Störungen des melatoninergischen Systems.

## Claims

1. Compound of formula (I) : 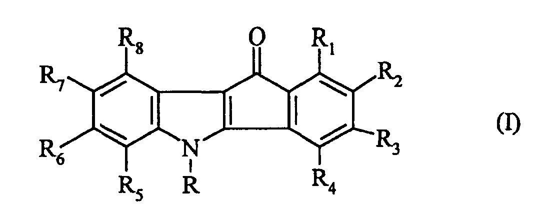 wherein:
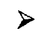 R represents a hydrogen atom or a linear or branched (C₁-C₆)alkenyl or linear or branched (C₁-C₆)alkyl group optionally substituted by a carboxy group or by a group of formula -NRₐR_{b} wherein each of Rₐ and R_{b}, which may be identical or different, represents a hydrogen atom or a linear or branched (C₁-C₆)alkyl group, or form together with the nitrogen atom carrying them a nitrogen-containing heterocycle,
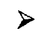 each of R₁, R₂, R₃, R₄, R₅, R₆, R₇ and R₈, which may be identical or different, represents a hydrogen atom or a linear or branched (C₁-C₆)alkyl group, a linear or branched (C₁-C₆)alkenyl group, a hydroxy group, a linear or branched (C₁-C₆)alkoxy group, a linear or branched (C₁-C₆)alkenyloxy group, an aryl-(C₁-C₆)alkyl group in which the alkyl moiety is linear or branched, an aryl-(C₁-C₆)alkoxy group in which the alkoxy moiety is linear or branched, a carboxy group, a linear or branched (C₁-C₆)-acyloxy group, or an arylcarbonyloxy group,
> or one of the groups R₁ to R₈ forms with an adjacent one of the groups R₁ to R₈ a (C₁-C₂)alkylenedioxy group,
its optical isomers when they exist, and addition salts thereof with a pharmaceutically acceptable acid or base,
with the proviso that:
* when R does not represent a hydrogen atom, then at least one of the groups R₁ to R₈ represents a hydroxy group or a linear or branched (C₁-C₆)acyloxy group,
* and the compounds of formula (I) are other than indeno[1,2-b]indol-10(5*H*)-one,
it being understood that the term "aryl group" is understood to mean "phenyl", "biphenylyl", "naphthyl" or "tetrahydronaphthyl", each of those groups being optionally substituted by one or more identical or different atoms or groups selected from halogen atoms and linear or branched (C₁-C₆)alkyl groups, hydroxy groups, linear or branched (C₁-C₆)alkoxy groups, linear or branched (C₁-C₆)polyhaloalkyl groups, amino groups (optionally substituted by one or more linear or branched (C₁-C₆)alkyl groups), nitro groups, linear or branched (C₁-C₆)acyl groups and (C₁-C₂)alkylenedioxy groups.

2. Compound of formula (I) according to claim 1 which is 2,3-dimethoxy-8-hydroxy-5-methyl-indeno[1,2-b]indol-10(5*H*)-one.

3. Compound of formula (I) according to claim 1 which is 2,4-dimethoxy-8-hydroxyindeno[1,2-b]indol-10(5*H*)-one.

4. Compound of formula (I) according to claim 1 which is 2,3-dimethoxy-5-(2-dimethylaminoethyl)-8-hydroxy-indeno[1,2-b]indol-10(5*H*)-one.

5. Compound of formula (I) according to claim 1 which is 2,3-dimethoxy-8-hydroxy-5-[2-(4-morpholinyl)-ethyl]-indeno[1,2-b]indol-10(5*H*)-one.

6. Process for the preparation of compounds of formula (I) according to claim 1, **characterised in that** a compound of formula (II) : 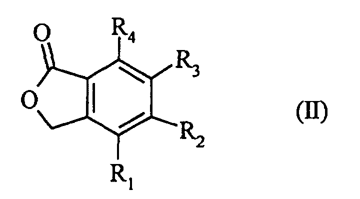 wherein R₁, R₂, R₃ and R₄ are as defined for formula (I),
is reacted with N-bromosuccinimide to yield a compound of formula (III) : 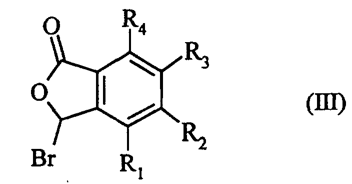 wherein R₁, R₂, R₃ and R₄ are as defined hereinbefore,
which is reacted with triphenylphosphine to yield a compound of formula (IV) : 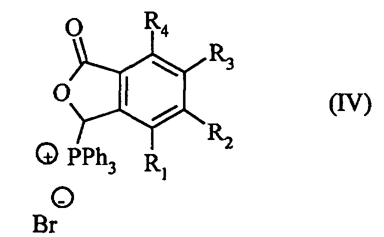 wherein R₁, R₂, R₃ and R₄ are as defined hereinbefore,
which is reacted with a compound of formula (V) : 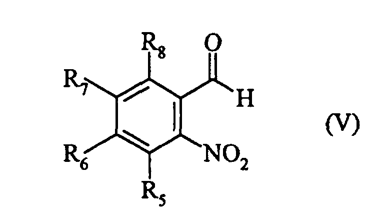 wherein R₅, R₆, R₇ and R₈ are as defined for formula (I),
to yield a compound of formula (VI) : 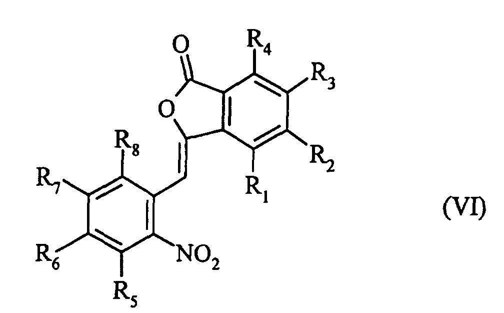 wherein R₁, R₂, R₃, R₄, R₅, R₆, R₇ and R₈ are as defined hereinbefore,
which is treated with a base to yield a compound of formula (VII) : 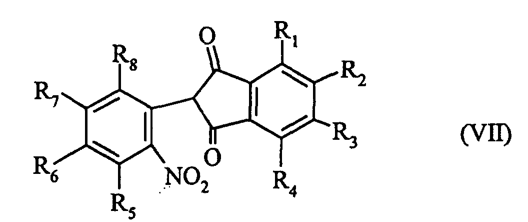 wherein R₁, R₂, R₃, R₄, R₅, R₆, R₇ and R₈ are as defined hereinbefore,
which is subjected to the action of a reducing agent to yield, after separation of the isomers where necessary, a compound of formula (Ia), a particular case of the compounds of formula (I) : 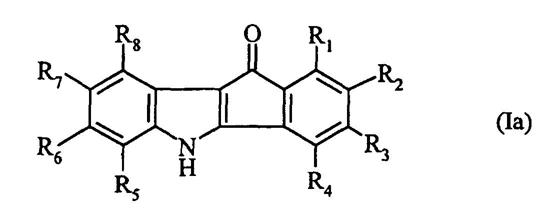 wherein R₁, R₂, R₃, R₄, R₅, R₆, R₇ and R₈ are as defined hereinbefore,
which is reacted, if desired, with a compound of formula (VIII):
R'-Z (VIII)
wherein R' represents a linear or branched (C₁-C₆)alkenyl or linear or branched (C₁-C₆)alkyl group optionally substituted by a carboxy group or by a group of formula -NRₐR_{b} wherein each of Rₐ and R_{b}, which may be identical or different, represents a hydrogen atom or a linear or branched (C₁-C₆)alkyl group, or form together with the nitrogen atom carrying them a nitrogen-containing heterocycle, and Z represents a leaving group, such as, for example, a halogen atom or a mesylate, tosylate or trifluoromethanesulphonate group,
to yield a compound of formula (Ib), a particular case of the compounds of formula (I) : 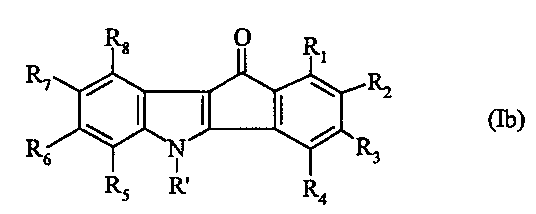 wherein R₁, R₂, R₃, R₄, R₅, R₆, R₇, R₈ and R' are as defined hereinbefore,
which compounds of formulae (Ia) and (Ib) constitute the totality of the compounds of formula (I), which are purified, if necessary, in accordance with a conventional purification technique, are separated, where appropriate, into their isomers in accordance with a conventional separation technique, and converted, if desired, into addition salts thereof with a pharmaceutically acceptable acid or base.

7. Pharmaceutical composition comprising as active ingredient a compound of formula (I) according to any one of claims 1 to 5, in combination with one or more inert, non-toxic pharmaceutically acceptable carriers.

8. Pharmaceutical composition according to claim 7 for use in the manufacture of medicaments for treating disorders of the melatoninergic system.
